# EUROPÄISCHE PATENTSCHRIFT

(11) **EP 3 172 037 B1**
(45) Veröffentlichungstag und Bekanntmachung des Hinweises auf die Patenterteilung: **08.07.2020**
(21) Anmeldenummer: 15742207.2
(22) Anmeldetag: 17.07.2015
(51) Int. Cl.: B29C 64/112, B29C 64/135, B29C 64/393, B33Y 10/00, B33Y 30/00

(54) **VERFAHREN ZUR HERSTELLUNG EINES BAUTEILS MITTELS EINES GENERATIVEN FERTIGUNGSPROZESSES, ANLAGE ZUR HERSTELLUNG EINES BAUTEILS MITTELS EINES GENERATIVEN FERTIGUNGSPROZESSES UND PATIENTENINDIVIDUELL ERZEUGTES MEDIZINISCHES IMPLANTAT**
METHOD FOR MANUFACTURING A COMPONENT BY A GENERATIVE MANUFACTURING PROCESS, APPARATUS FOR MANUFACTURING A COMPONENT BY A GENERATIVE MANUFACTURING PROCESS, AND MEDICAL IMPLANT GENERATED FOR AN INDIVIDUAL PATIENT
PROCÉDÉ DE FABRICATION D'UN COMPOSANT AU MOYEN D'UN PROCÉDÉ DE FABRICATION GÉNÉRATIF, INSTALLATION POUR LA FABRICATION D'UN COMPOSANT AU MOYEN D'UN PROCÉDÉ DE FABRICATION GÉNÉRATIF ET IMPLANT MÉDICAL GÉNÉRÉ DE MANIÈRE INDIVIDUELLE À UN PATIENT

(30) Priorität: 22.07.2014 DE 102014010677; 09.02.2015 DE 102015101810
(43) Veröffentlichungstag der Anmeldung: 31.05.2017
(73) Patentinhaber: Medizinische Hochschule Hannover, 30625 Hannover (DE)
(72) Erfinder: STIEGHORST, Jan, 29352 Adelheidsdorf (DE); DOLL, Theodor, 44894 Bochum (DE)
(74) Vertreter: Gramm, Lins & Partner Patent- und Rechtsanwälte PartGmbB
(86) Internationale Anmeldenummer: PCT/EP2015/066441
(87) Internationale Veröffentlichungsnummer: WO 2016/012370

(56) Entgegenhaltungen:
- US-A1- 2009 014 916
- US-A1- 2010 208 006

## Beschreibung

Die Erfindung betrifft ein Verfahren zur Herstellung eines Bauteils mittels eines generativen Fertigungsprozesses. Die Erfindung betrifft ferner eine Anlage zur Herstellung eines Bauteils mittels eines generativen Fertigungsprozesses gemäß Anspruch 11. Insbesondere betrifft die Erfindung das Gebiet der generativen Fertigung von Bauteilen aus flüssigem, durch Erwärmung verfestigbarem Rohmaterial, z.B. für die medizinisches Implantatversorgung mit individualisierten Implantaten.

Generative Fertigungsprozesse sind z.B. Stereolithografie, selektives Laserschmelzen, selektives Lasersintern, Fused Deposition Modeling, Laminated Object Modelling, 3D-Printing (auch 3D-Druck genannt) sowie Kaltgasspritzen, insbesondere auch sämtliche Fertigungsverfahren des Rapid Prototyping, des Rapid Tooling und des Rapid Manufacturing.

Im Gebiet der generativen Fertigung aus flüssigen Kunststoffen sind das Poly-Jet Modeling, das Digital Light Processing und die Stereolithographie bekannt, wobei die Stereolithographie gegenwärtig die genaueste aller generativen Fertigungsverfahren ist (J. Breuninger, R. Becker, A. Wolf, S. Rommel und A. Verl, Generative Fertigung mit Kunststoffen, Springer-Verlag Berlin Heidelberg, 27-28 (2013)). Grundlage dieser Verfahren ist der schichtweise Auftrag und die schichtweise strukturierende Vernetzung eines photosensitiven Polymers, welches durch UV-Strahlung vernetzt wird. Angewendet werden diese Verfahren insbesondere im Prototypenbau, wobei erste Verfahren zur Serienfertigung in der Umsetzung sind. Im Bereich der medizinischen Implantatversorgung werden generative Verfahren unter dem Begriff "Medical Rapid Prototyping" zusammengefasst. Die Verfahren werden zur Fertigung dimensionaler Modelle von menschlichen anatomischen Strukturen verwendet, die auf der Grundlage medizinischer Bildgebung erstellt werden. Gegenwärtig werden Medical Rapid Prototyping-Verahren unter anderem zur Versorgung von Hartgewebedefekten, wie zum Beispiel Schädeldefekten angewendet, bei der mit Hilfe von Elektrodenstrahlschmelz- oder Lasersinter-Verfahren direkt angepasste metallische Schädelplatten produziert werden können (A. D. Lantada und P. L. Morgado, Annu. Rev. Biomed. Eng. 14, 73-96 (2012)). Im Bereich der Weichgewebeimplantate ist im Gegensatz dazu keine direkte generative Herstellung von medizinischen Implantaten möglich, da die hier verwendeten Polymere gegenwärtig nur von Gussformen abgeformt und nicht direkt gedruckt werden können.

Aus der US 2009/014916 A1 sind ein Verfahren und eine Einrichtung zur Herstellung einer dreidimensionalen Struktur gemäß den Oberbegriffen der Ansprüche 1 und 11 bekannt. Aus der US 2010/208006 A1 ist das Drucken von bioreaktiven Materialien bekannt.

Der Erfindung liegt daher die Aufgabe zugrunde, ein Verfahren und eine Anlage zur Herstellung eines Bauteils mittels eines generativen Fertigungsprozesses anzugeben, bei dem als zu verarbeitendes Material ein zunächst flüssiges Rohmaterial verwendet werden kann.

Diese Aufgabe wird durch ein Verfahren gemäß Anspruch 1 gelöst.

Durch die Erfindung wird ein wesentliches Problem gelöst, das bei der Verwendung flüssigen Rohmaterials ohne Zuhilfenahme einer Negativform auftritt, nämlich dass das Rohmaterial relativ schnell von der Auftragstelle wegfließt bzw. auseinanderfließt. Die Erfindung löst dies durch rechnergesteuerte, punktuelle gezielte Lichtbestrahlung sowie dadurch, dass als Rohmaterial ein durch Erwärmung verfestigbares Rohmaterial eingesetzt wird. Hierdurch kann das flüssig aufgetragene Rohmaterial gezielt relativ schnell zumindest soweit ausgehärtet werden, dass es seine Form beibehält und nicht mehr wegfließt.

Ein weiterer Vorteil ist, dass Rohmaterialien verwendet werden können, die nicht durch Ultraviolett-Lichtbestrahlung auszuhärten sind, d.h. die keine UV-Vernetzer aufweisen. Hierdurch können in vielen Anwendungsfällen besser verträgliche Bauteile hergestellt werden, insbesondere medizinische Implantate. Als Rohmaterial können insbesondere bereits zugelassene medizinische Materialien verwendet werden.

Die eingangs genannte Problematik bei der Verwendung flüssigen Rohmaterials wiegt in der Herstellung von flexiblen Implantaten schwer, da eine Fertigung individuell angepasster Polymer-basierter Implantate bisher nicht möglich erschien. Es ist gewünscht, dass feste sowie auch flexible Implantate für eine optimale Wirkung möglichst gezielt an den individuellen Zielort jedes einzelnen Patienten angepasst und positioniert werden sollten. Ist dies nicht gegeben, so kann das Implantat im ungünstigsten Fall nicht nur seine angedachte Funktion nicht erfüllen, sondern auch gesunde Strukturen und Abläufe schädigen. Eine Herzschrittmacher-Elektrode, ein Elektrodenschaft eines Cochlea-Implantats, eine Sonde zur tiefen Hirnstimulation oder ein Implantat zur kortikalen Ableitung wären wenig nutzbringend, wenn ihre Elektrodenkontakte nicht in unmittelbarer Umgebung zu den anvisierten Zellen positioniert würden. Ideal wäre also ein Verfahren, welches zusätzlich zur Herstellung anatomisch angepasster Implantate aus festen Materialien auch die Herstellung aus flexiblen Materialien ermöglicht. Weiterhin sollten bereits verfügbare Werkstoffe verarbeitet werden können, da mit den oben genannten Verfahren nur photosensitive Kunststoffe verarbeitet werden können. Dies grenzt insbesondere in der Medizintechnik die Materialauswahl stark ein, da photosensitive und gleichzeitig biokompatible Kunststoffe rar sind. Gängige Materialien wie zum Beispiel Silikonelastomere (Anwendung in Brustimplantaten, Cochlea-Implantatsystemen, Sonden, Herschrittmachern, usw.) können bei der Verwendung von UV-Vernetzern, nur in Kurzzeitanwendungen verwendet werden, da die UV-Vernetzer nicht für Langzeitanwendungen zugelassen sind. Im Gegensatz dazu sind wärmevernetzende Silikonelastomere erprobt und für die Langzeitanwendung bereits zugelassen.

Wie bereits erwähnt, wird der Auftreffpunkt des Lichtstrahls der Lichtstrahlquelle relativ zum Fertigungsbereich kontinuierlich und/oder schrittweise verändert. Dies kann z.B. dadurch erfolgen, dass die Lichtstrahlquelle hinsichtlich der Lichtabstrahlrichtung steuerbar ist, so dass die Abstrahlrichtung des Lichtstrahls kontinuierlich und/oder schrittweise verändert wird und damit unterschiedliche Stellen des Fertigungsbereiches bestrahlt werden. Alternativ oder in Kombination damit kann auch der Fertigungsbereich positionsveränderbar sein, z.B. in Form eines in wenigstens zwei Raumrichtungen verfahrbaren Tischs, auf dem das generativ zu fertigende Bauteil aufgebaut wird.

Der Fertigungsbereich kann insbesondere ein Fertigungsbereich einer Anlage zur Herstellung eines Bauteils mittels eines generativen Fertigungsprozesses sein, z.B. eine ebene oder gewölbte Fläche, auf der das Rohmaterial abgegeben wird und wo das Bauteil dann generativ entsteht.

Gemäß der Erfindung weist das verfestigte Rohmaterial weiterhin eine hohe Elastizität auf. Dies hat den Vorteil, dass mit der Erfindung flexible bzw. hochelastische Bauteile hergestellt werden können, wie z.B. Weichgewebeimplantate für den medizinischen Bereich. Das verfestigte Rohmaterial kann insbesondere ein gummielastisches Material sein. Gemäß einer vorteilhaften Weiterbildung der Erfindung weist das verfestigte Rohmaterial eine Shore-Härte im Bereich von 10 bis 90 Shore-A auf, insbesondere 20 bis 60 Shore-A.

Das Rohmaterial kann insbesondere ein Polymermaterial sein. In diesem Fall wird das Rohmaterial im flüssigen Zustand auch als Präpolymer bezeichnet. Insbesondere kann das Rohmaterial ein Silikonmaterial sein, z.B. Silikonkautschuk. Für die Herstellung medizinischer Implantate sind insbesondere biokompatible, für den medizinischen Bereich zugelassene Silikone obligatorisch.

Gemäß einer vorteilhaften Weiterbildung der Erfindung ist der Lichtstrahl der Lichtstrahlquelle als fein fokussierter Lichtstrahl ausgebildet. Insbesondere kann der Lichtstrahl einen Durchmesser ? 300 µm, noch besser ? 200 µm, am Auftreffpunkt auf dem zu verfestigenden Rohmaterial aufweisen. Dies erlaubt die Herstellung des Bauteils mit einer hohen dreidimensionalen Auflösung, so dass auch fein detaillierte Bauteile hergestellt werden können.

Gemäß einer vorteilhaften Weiterbildung der Erfindung wird der Auftreffpunkt des Lichtstrahls der Lichtstrahlquelle relativ zum Fertigungsbereich einer Rohmaterial-Abgabeeinrichtung, die tropfenweise das Rohmaterial abgibt, während des Fertigungsprozesses des Bauteils nachgeführt, so dass frisch von der Rohmaterial-Abgabeeinrichtung abgegebene Rohmaterial-Tropfen sogleich durch den Lichtstrahl erwärmt und dadurch verfestigt werden. Dies hat den Vorteil, dass das Bauteil durch punktweisen Auftrag des Rohmaterials quasi in jeder beliebigen Formgebung besonders zügig hergestellt werden kann. Durch das nach Abgabe eines Rohmaterial-Tropfens kurzfristig durch den Lichtstrahl erzeugte Erwärmen wird dieser Tropfen gleich soweit verfestigt, dass er nicht abfließen kann. Das Nachführen des Auftreffpunkts des Lichtstrahls mit der Rohmaterial-Abgabeeinrichtung kann beispielsweise derart realisiert werden, dass die Lichtstrahlquelle fest mit der Rohmaterial-Abgabeeinrichtung mechanisch gekoppelt ist und die Abstrahlrichtung des Lichtstrahls entsprechend justiert ist, so dass automatisch der Lichtstrahl auf einen frisch von der Rohmaterial-Abgabeeinrichtung abgegebenen Rohmaterial-Tropfen auftrifft. Es ist auch möglich, dass die Lichtstrahlquelle und die Rohmaterial-Abgabeeinrichtung voneinander mechanisch entkoppelt sind. In diesem Fall ist es vorteilhaft, die Abstrahlrichtung des Lichtstrahls der Lichtstrahlquelle rechnergesteuert der Rohmaterial-Abgabeeinrichtung nachzuführen.

Gemäß der Erfindung wird das flüssige Rohmaterial in der Rohmaterial-Abgabeeinrichtung mittels einer Vorerwärmungseinrichtung bereits vor der Abgabe vorerwärmt und damit zum Teil verfestigt. Dies hat den Vorteil, dass die Viskosität des flüssigen Rohmaterials bereits vorab etwas erhöht werden kann (Vorvulkanisation) und damit der nach Abgabe des Rohmaterials aus der Rohmaterial-Abgabeeinrichtung durch den Lichtstrahl zu erzeugende Energieeintrag verringert wird. Hierdurch kann die Arbeitsgeschwindigkeit und Präzision des generativen Fertigungsprozesses erhöht werden.

Gemäß einer vorteilhaften Weiterbildung der Erfindung wird das Rohmaterial mit einem aushärtungsfördernden Material gemischt. Dies kann z.B. vorab geschehen, d.h. bevor das Rohmaterial in dem generativen Fertigungsprozess eingesetzt wird, z.B. herstellerseitig, oder während des generativen Fertigungsprozesses. So kann z.B. das Mischen mit dem aushärtungsfördernden Material innerhalb der Rohmaterial-Abgabeeinrichtung über einen dort angeordneten Mischer erfolgen. Das Beimischen eines aushärtungsfördernden Materials hat den Vorteil, dass der von der Lichtstrahlquelle zu erzeugende Energieeintrag zum Verfestigen des flüssigen Rohmaterials verringert wird. Dies ist ebenfalls vorteilhaft für eine hohe Arbeitsgeschwindigkeit und Präzision des generativen Fertigungsprozesses.

Gemäß einer vorteilhaften Weiterbildung der Erfindung werden als aushärtungsförderndes Material lichtabsorbierende Partikel zugemischt, durch die die Lichtabsorption der auf die Weise gebildeten Masse erhöht wird und die damit die Verfestigung durch Lichtbestrahlung beschleunigt wird.

Gemäß einer vorteilhaften Weiterbildung der Erfindung ist der Lichtstrahl ein Infrarot-Lichtstrahl oder weist wenigstens überwiegend Infrarot-Spektralanteile auf. Dies hat den Vorteil, dass mittels der Lichtstrahlquelle eine hohe Erwärmungseffizienz in dem zu verfestigenden Rohmaterial erzielt werden kann. Die Lichtstrahlquelle kann z.B. eine Laserlichtquelle sein, z.B. eine Infrarot-Laserdiode, ein Kohlenmonoxid-Laser oder ein CO2-Laser. Geeignet ist ferner ein Keramik-Infrarotemitter. Der gewünschte, fein fokussierte Lichtstrahl der Lichtstrahlquelle kann auch über eine geeignete Optik, z.B. ein Linsensystem, erzeugt werden.

Gemäß einer vorteilhaften Weiterbildung der Erfindung wird das Bauteil mit mehreren funktionalen Schichten hergestellt, indem jeweilige Schichten durch das Auftragen weiteren Rohmaterials auf bereits verfestigte Bereiche des Bauteils aufgetragen werden, die wiederum durch rechnergesteuerte gezielte Lichtbestrahlung verfestigt werden. Das Auftragen der weiteren Schichten kann, wie zuvor bereits erläutert, ebenfalls punktweise erfolgen.

Gemäß einer vorteilhaften Weiterbildung der Erfindung wird das Bauteil aus mehreren unterschiedlichen Materialien hergestellt, wobei aus dem durch Lichtbestrahlung verfestigten Rohmaterial zumindest eine Haltestruktur für wenigstens ein weiteres Teilelement des Bauteils erzeugt wird. Auf diese Weise können auch komplexe technische Bauteile erzeugt werden, insbesondere auch Bauteile mit elektrisch leitenden Strukturen. Auf diese Weise können mittels der Erfindung z.B. subdurale Elektrodenanordnungen erzeugt werden. Hierbei müssen die weiteren verwendeten Materialien, die außer dem flüssigen Rohmaterial eingesetzt werden, nicht zwangsläufig in demselben generativen Fertigungsprozess miterzeugt werden wie das eigentliche Bauteil, sondern sie können auch als vorgefertigte Teile bereitgestellt werden, z.B. in Form von Metallgitteranordnungen.

Gemäß einer vorteilhaften Weiterbildung der Erfindung wird das Rohmaterial mittels der rechnergesteuerten, punktuellen gezielten Lichtbestrahlung in einem ersten Arbeitsgang nicht vollständig verfestigt. Dies hat den Vorteil, dass der Fertigungsprozess weiter beschleunigt werden kann. Die Verweildauer des Lichtstrahls an einem bestimmten Rohmaterial-Punkt kann hierdurch reduziert werden. Ein weiterer Vorteil ist, dass das Rohmaterial infolge seiner nicht vollständigen Verfestigung Eigenschaften aufweisen kann, die günstig für die Haftung einer weiteren aufgetragenen Schicht des Rohmaterials sind.

Nach Durchführung des genannten ersten Arbeitsganges kann eine weitere bzw. endgültige Verfestigung des Rohmaterials in einem zweiten Arbeitsgang erfolgen. Dies kann z.B. durch pauschales Erwärmen des ansonsten bereits fertig hergestellten Bauteils erfolgen, z.B. in einem Ofen, oder durch erneute punktuelle Lichtbestrahlung, wobei wiederum der Auftreffpunkt des Lichtstrahls relativ zum Fertigungsbereich bzw. zum dann schon vorliegenden Bauteil verändert wird. In dem zweiten Arbeitsgang kann der Lichtstrahl weniger fein fokussiert sein, d.h. einen größeren Durchmesser im Auftreffpunkt aufweisen als im ersten Arbeitsgang.

Gemäß einer vorteilhaften Weiterbildung der Erfindung wird das Bauteil nach vollständigem oder teilweisem Abschluss des Verfestigungsprozesses des flüssigen Rohmaterials in dem zweiten Arbeitsgang durch rechnergesteuerte, gezielte Lichtbestrahlung einer Wärmenachbehandlung unterzogen.

Der Fertigungsbereich kann insbesondere eine Plattform aufweisen, auf der das Bauteil erzeugt wird. So kann das Bauteil durch rechnergesteuerten punktweisen Auftrag des flüssigen Rohmaterials auf der Plattform und/oder auf bereits verfestigtem Rohmaterial und anschließender Verfestigung durch Lichtbestrahlung eines jeweiligen noch flüssigen Rohmaterial-Punkts hergestellt werden.

Gemäß einer vorteilhaften Weiterbildung der Erfindung werden einer oder mehrere flüssige Rohmaterial-Punkte auf der Plattform und/oder auf bereits verfestigtem Rohmaterial aufgetragen und mit Lichtbestrahlung verfestigt, bevor weitere Punkte flüssigen Rohmaterials aufgetragen werden. Hierdurch wird auch einem Wegfließen des flüssigen Rohmaterials entgegengewirkt, indem es punktweise zügig erwärmt und damit verfestigt wird.

Gemäß einer vorteilhaften Weiterbildung der Erfindung wird das flüssige Rohmaterial schichtweise auf einer Plattform und/oder auf bereits verfestigtem Rohmaterial aufgetragen und durch rechnergesteuerte, gezielte Lichtbestrahlung verfestigt, bevor eine weitere Schicht flüssigen Rohmaterials aufgebracht wird. Im Gegensatz zum punktweisen Auftragen des Rohmaterials hat das schichtweise Auftragen den Vorteil, dass die Arbeitsgeschwindigkeit des Fertigungsprozesses weiter erhöht werden kann. Überschüssiges, nicht durch den Lichtstrahl verfestigtes Rohmaterial kann dann später entfernt werden.

Gemäß einer vorteilhaften Weiterbildung der Erfindung wird die Plattform nach dem Auftrag von einer oder mehreren Schichten des Rohmaterials jeweils abgesenkt. Auf diese Weise muss die Rohmaterial-Abgabeeinrichtung nicht in allen drei Raumrichtungen bewegbar ausgeführt sein, sondern kann z.B. nur in einer Ebene, d.h. in zwei Raumrichtungen bewegbar sein. Hierdurch vereinfacht sich die Mechanik einer Anlage zur Herstellung des Bauteils.

Gemäß einer vorteilhaften Weiterbildung der Erfindung wird das flüssige Rohmaterial durch mehrere Lichtstrahlen verfestigt, die sich in einem Punkt des Rohmaterials kreuzen, der verfestigt werden soll. Dies hat den Vorteil, dass andere Bereiche des Rohmaterials, die bereits verfestigt sind, nicht unerwünscht erwärmt werden. Zudem kann auch hierdurch die Arbeitsgeschwindigkeit des generativen Fertigungsprozesses erhöht werden. Die sich kreuzenden Lichtstrahlen können z.B. von zwei separat rechnergesteuerten Lichtstrahlquellen abgegeben werden.

Gemäß einer vorteilhaften Weiterbildung der Erfindung wird in einem oder mehreren Bereichen des herzustellenden Bauteils dem Rohmaterial im flüssigen Zustand elektrisch leitfähiges Material zugemischt. Auf diese Weise können bestimmte Bereich des hergestellten Bauteils als elektrisch leitfähige Bereiche ausgebildet werden. Das elektrisch leitfähige Material wird durch das Rohmaterial im verfestigten Zustand an einer definierten Position im hergestellten Bauteil gehalten. Als elektrisch leitfähiges Material können z.B. carbon nanotubes verwendet werden. Das Mischen mit dem elektrisch leitfähiges Material kann innerhalb der Rohmaterial-Abgabeeinrichtung über einen dort angeordneten Mischer erfolgen

Gemäß einer vorteilhaften Weiterbildung der Erfindung wird die Lichtabgabe der Lichtstrahlquelle auf das Rohmaterial mittels eines Shutters ein- und ausgeschaltet. Dies hat den Vorteil, dass auch solche Lichtstrahlquellen eingesetzt werden können, deren Ein- und Ausschaltevorgang mit relativ großem Totzeiten behaftet ist, wie z.B. elektrisch aufgeheizte Glühwendel. Bei Verwendung von Laserdioden als Lichtstrahlquelle wird ein solcher Shutter häufig nicht erforderlich sein, kann aber dennoch auch in solchen Fällen vorteilhaft eingesetzt werden.

Gemäß einer vorteilhaften Weiterbildung der Erfindung wird das Bauteil durch Steuerung der gezielten Lichtbestrahlung mit bereichsweise unterschiedlichen Elastizitätswerten des verfestigten Materials hergestellt. Dies hat den Vorteil, dass ein Bauteil aus ein und demselben Rohmaterial hergestellt werden kann, das letztendlich eine gewünschte inhomogene Elastizitätsverteilung aufweist. Hierbei kann insbesondere die materialspezifische Elastizität in unterschiedlichen Bereichen des Bauteils variieren.

Die eingangs genannte Aufgabe wird ferner gelöst durch eine Anlage gemäß Anspruch 11.

Mit einer solchen Anlage können ebenfalls die zuvor bezüglich des Verfahrens genannten Vorteile realisiert werden. Auch die zuvor erläuterten einrichtungsmäßigen Weiterbildungen der Erfindung sind auch als vorteilhafte Weiterbildungen der Anlage realisierbar. Insbesondere kann die Anlage einen Shutter aufweisen. Der Shutter kann durch die Steuereinheit steuerbar sein. Ferner kann die Anlage mehrere hinsichtlich der Abstrahlrichtung steuerbare Lichtstrahlquellen aufweisen. Die Lichtstrahlquellen können hinsichtlich ihrer Abstrahlrichtung und/oder hinsichtlich der Lichtabgabe (Helligkeit bzw. Ein-/Ausschalten der Lichtabgabe) von der Steuereinheit steuerbar sein. Die Lichtstrahlquelle kann eine Infrarot-Lichtstrahlquelle sein oder wenigstens überwiegend Infrarot-Spektralanteile im abgegebenen Licht aufweisen. Insbesondere kann die Lichtstrahlquelle als Laserlichtquelle ausgebildet sein.

Offenbart wird ferner ein patientenindividuell erzeugtes medizinisches Implantat aus gummielastischem Material ohne stabilisierende Gerüststruktur, mit einer oder mehreren im gummielastischem Material eingebetteten Elektroden, die zumindest an einer jeweiligen Außenkontaktfläche nicht mit dem gummielastischen Material überdeckt sind. Ein solches medizinisches Implantat wird generativ hergestellt, und zwar mit einem Verfahren mittels eines generativen Fertigungsprozesses der zuvor erläuterten Art und/oder mit einer Anlage der zuvor erläuterten Art. Auf diese Weise lassen sich effizient, präzise und schnell patientenindividuelle medizinische Implantate bereitstellen. Das medizinische Implantat weist aufgrund der generativen Herstellung eine Pixelstruktur auf. Diese ist aber bei entsprechend hoher Auflösung des generativen Fertigungsprozesses, d.h. bei Verwendung eines fein fokussierten Lichtstrahls und entsprechend kleiner Auftreffpunkte des Lichtstrahls auf dem zu verfestigenden Rohmaterial praktisch nicht wahrnehmbar, zumindest nicht störend. Das gummielastische Material des medizinischen Implantats kann eine Shore-Härte im Bereich von 10 bis 90 Shore-A, insbesondere 20 bis 60 Shore-A, aufweisen. Das Implantat kann mehrschichtig mit mehreren funktionalen Schichten ausgebildet sein.

Gemäß einer vorteilhaften Weiterbildung ist das Implantat als ECoG-Elektrodenanordnung, als Herzschrittmacher-Elektrodenanordnung, als Cochlea-Implantat-Elektrodenanordnung oder als Hirnstamm-Implantat-Elektrodenanordnung ausgebildet.

Gemäß einer vorteilhaften Weiterbildung sind eine, mehrere oder alle Elektroden des Implantats durch lose in das gummielastische Material des Implantats eingemischtes elektrisch leitfähiges Material gebildet.

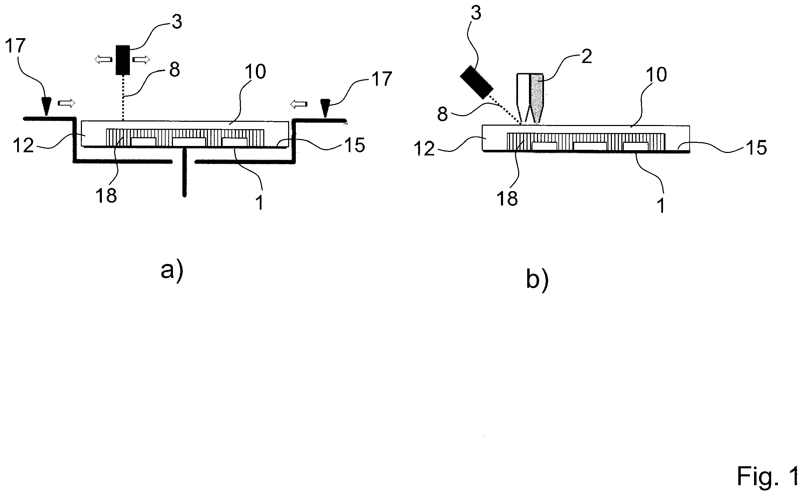

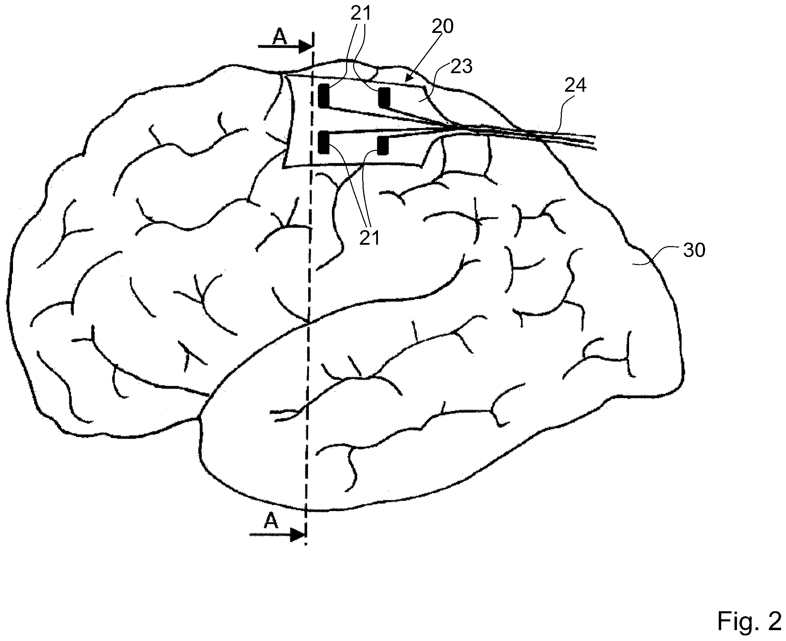

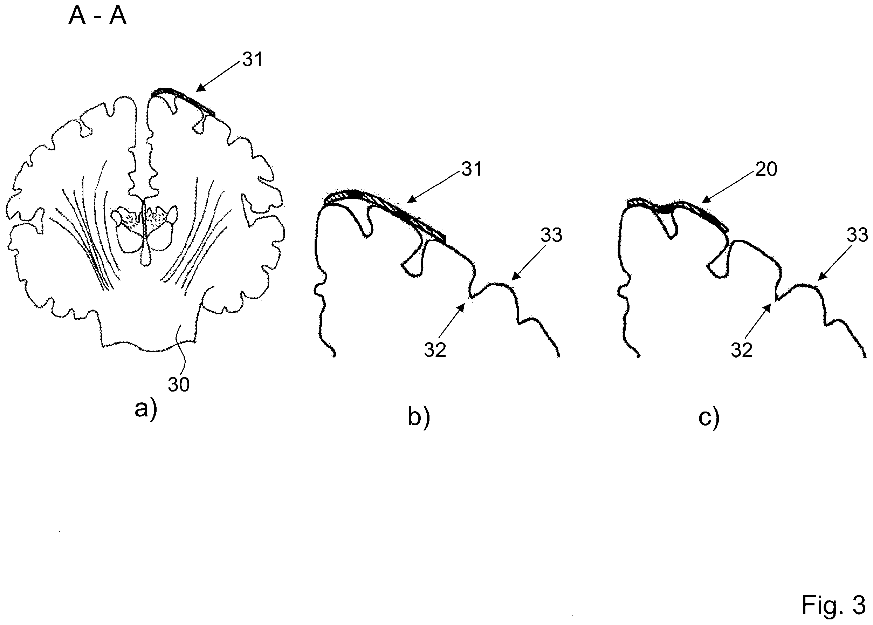

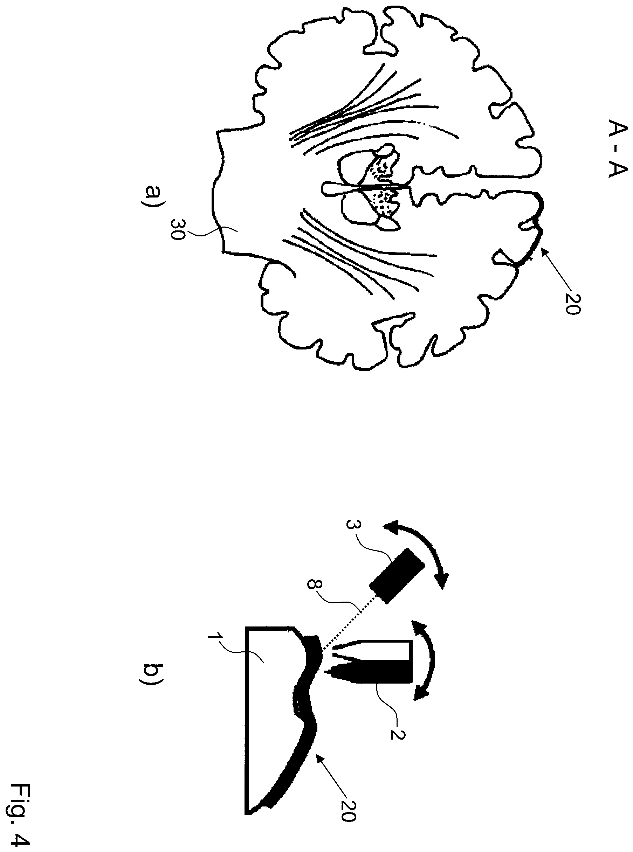

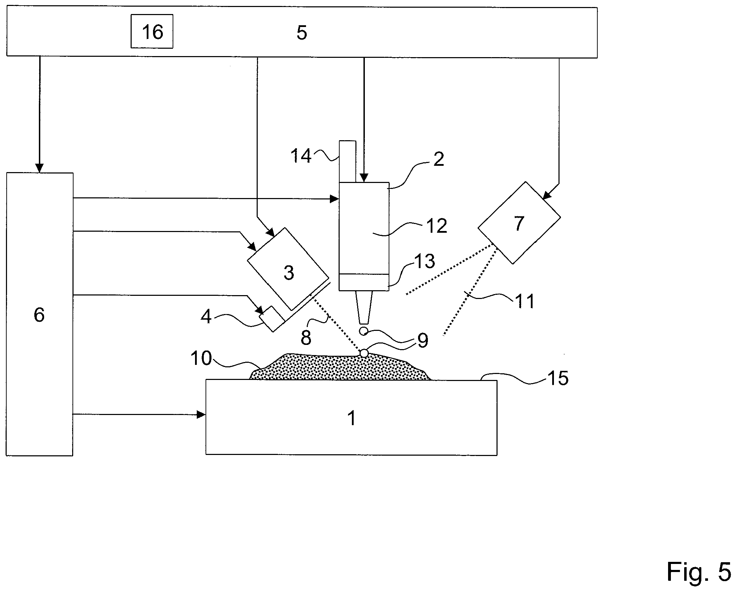

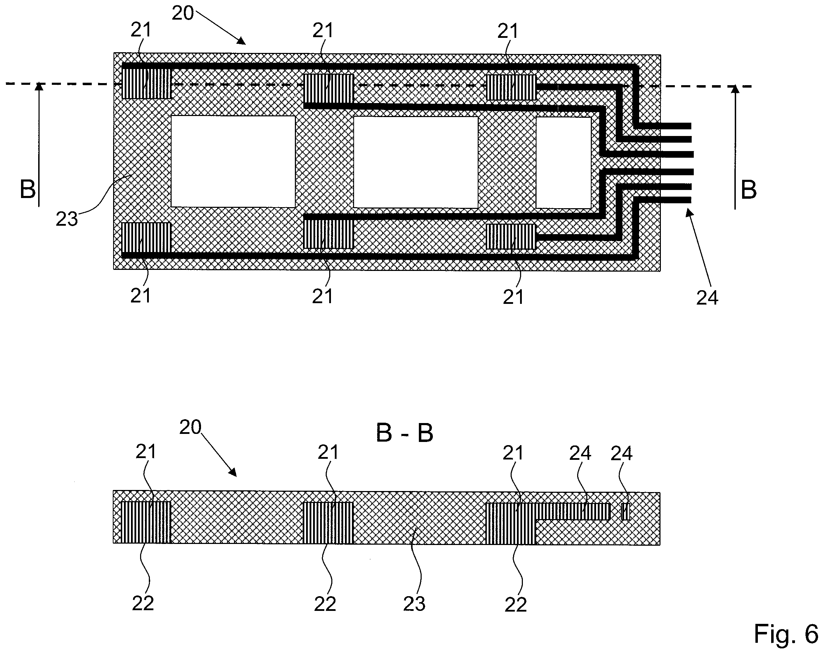

## Patentansprüche

1. Verfahren zur Herstellung eines Bauteils (10) mittels eines generativen Fertigungsprozesses, wobei das Bauteil (10) ganz oder teilweise aus einem flüssigen Rohmaterial (12) hergestellt wird, wobei das Bauteil (10) ganz oder teilweise aus einem flüssigen, durch Erwärmung verfestigbaren Rohmaterial (12) hergestellt wird, wobei das Rohmaterial (12) in flüssiger Form in einen Fertigungsbereich (1) abgegeben wird und durch rechnergesteuerte, punktuelle gezielte Lichtbestrahlung erwärmt und dadurch verfestigt wird, indem der Auftreffpunkt eines Lichtstrahls (8) einer Lichtstrahlquelle (3) relativ zum Fertigungsbereich (1) kontinuierlich und/oder schrittweise verändert wird, **dadurch gekennzeichnet, dass** das flüssige Rohmaterial (12) in der Rohmaterial-Abgabeeinrichtung mittels einer Vorerwärmungseinrichtung (13) bereits vor der Abgabe vorerwärmt und damit zum Teil verfestigt wird.

2. Verfahren nach einem der vorhergehenden Ansprüche, **dadurch gekennzeichnet, dass** das Rohmaterial (12) ein Polymermaterial ist

3. Verfahren nach einem der vorhergehenden Ansprüche, **dadurch gekennzeichnet, dass** der Auftreffpunkt des Lichtstrahls (8) der Lichtstrahlquelle (3) relativ zum Fertigungsbereich (1) einer Rohmaterial-Abgabeeinrichtung (2), die tropfenweise das Rohmaterial (12) abgibt, während des Herstellungsprozesses des Bauteils (10) nachgeführt wird, so dass frisch von der Rohmaterial-Abgabeeinrichtung (2) abgegebene Rohmaterial-Tropfen (9) sogleich durch den Lichtstrahl (8) erwärmt und dadurch verfestigt werden.

4. Verfahren nach einem der vorhergehenden Ansprüche, **dadurch gekennzeichnet, dass** das Rohmaterial (12) mit einem aushärtungsfördernden Material gemischt wird.

5. Verfahren nach einem der vorhergehenden Ansprüche, **dadurch gekennzeichnet, dass** der Lichtstrahl (8) ein Infrarot-Lichtstrahl ist oder wenigstens überwiegend Infrarot-Spektralanteile aufweist.

6. Verfahren nach einem der vorhergehenden Ansprüche, **dadurch gekennzeichnet, dass** das Bauteil (10) aus mehreren unterschiedlichen Materialien hergestellt wird, wobei aus dem durch Lichtbestrahlung verfestigten Rohmaterial (12) zumindest eine Haltestruktur für wenigstens ein weiteres Teilelement des Bauteils (10) erzeugt wird.

7. Verfahren nach einem der vorhergehenden Ansprüche, **dadurch gekennzeichnet, dass** das Rohmaterial (12) mittels der rechnergesteuerten, punktuellen gezielten Lichtbestrahlung in einem ersten Arbeitsgang nicht vollständig verfestigt wird.

8. Verfahren nach dem vorhergehenden Anspruch, **dadurch gekennzeichnet, dass** einer oder mehrere flüssige Rohmaterialpunkte (9) auf einer Plattform (15) und/oder auf bereits verfestigtem Rohmaterial aufgetragen werden und mit Lichtbestrahlung verfestigt werden, bevor weitere Punkte (9) flüssigen Rohmaterials (12) aufgetragen werden.

9. Verfahren nach einem der vorhergehenden Ansprüche, **dadurch gekennzeichnet, dass** das flüssige Rohmaterial (12) schichtweise auf einer Plattform (15) und/oder bereits verfestigtem Rohmaterial aufgetragen wird und durch rechnergesteuerte, gezielte Lichtbestrahlung verfestigt wird, bevor eine weitere Schicht flüssigen Rohmaterials (12) aufgebracht wird.

10. Verfahren nach einem der vorhergehenden Ansprüche, **dadurch gekennzeichnet, dass** das Bauteil (10) nach vollständigem oder teilweisem Abschluss des Verfestigungsprozesses des flüssigen Rohmaterials (12) durch rechnergesteuerte, gezielte Lichtbestrahlung einer Wärmenachbehandlung unterzogen wird.

11. Anlage zur Herstellung eines Bauteils (10) mittels eines generativen Fertigungsprozesses, aufweisend:
a) einen Fertigungsbereich (1), an dem das herzustellende Bauteil (10) mittels dort anzuordnenden flüssigen, durch Erwärmung verfestigbaren Rohmaterials (12) entsteht,
b) eine Rohmaterial-Abgabeeinrichtung (2) zur Abgabe des Rohmaterials (12) an den Fertigungsbereich (1),
c) eine Lichtstrahlquelle (3) zur Abgabe eines Lichtstrahls (8),
d) einen steuerbaren Aktuatormechanismus (6), mit dem der Auftreffpunkt des Lichtstrahls (8) der Lichtstrahlquelle (3) relativ zum Fertigungsbereich (1) kontinuierlich und/oder schrittweise veränderbar ist,
e) eine Steuereinheit (5) mit einem Rechner (16), die zur Steuerung des Aktuatormechanismus (6) eingerichtet ist,
f) wobei die Anlage zur Durchführung eines Verfahrens zur Herstellung eines Bauteils (10) mittels eines generativen Fertigungsprozesses nach einem der vorhergehenden Ansprüche eingerichtet ist,
**dadurch gekennzeichnet, dass** die Anlage eine Vorerwärmungseinrichtung (13) aufweist, durch die das flüssige Rohmaterial (12) in der Rohmaterial-Abgabeeinrichtung bereits vor der Abgabe vorerwärmt und damit zum Teil verfestigt wird.

## Claims

1. A method for producing a component (10) by means of an additive manufacturing process, wherein the component (10) is entirely or partly produced from a liquid raw material (12), wherein the component (10) is entirely or partly produced from a liquid raw material (12) solidifiable by heating, wherein the raw material (12) is dispensed in liquid form into a manufacturing zone (1) and is, as a result of computer-controlled, punctual targeted light irradiation, heated and thereby solidified, by the impact point of a light beam (8) of a light-beam source (3) relative to the manufacturing zone (1) being altered in a continuous and/or in a step-by-step manner, **characterized in that** the liquid raw material (12) in the raw material dispensing unit is already preheated before dispensing and thus partially solidified by means of a preheating unit (13).

2. The method as claimed in any of the preceding claims, **characterized in that** the raw material (12) is a polymer material.

3. The method as claimed in either of the preceding claims, **characterized in that** the impact point of the light beam (8) of the light-beam source (3) relative to the manufacturing zone (1) is, during the manufacturing process for the component (10), repositioned in relation to a raw material dispensing unit (2) which dispenses the raw material (12) in a dropwise manner, and so raw material drops (9) freshly dispensed by the raw material dispensing unit (2) are immediately heated and thereby solidified by the light beam (8).

4. The method as claimed in any of the preceding claims, **characterized in that** the raw material (12) is mixed with a curing-promoting material.

5. The method as claimed in any of the preceding claims, **characterized in that** the light beam (8) is an infrared light beam or has at least predominantly infrared spectral components.

6. The method as claimed in any of the preceding claims, **characterized in that** the component (10) is produced from multiple different materials, with at least one holding structure for at least one further subelement of the component (10) being generated from the raw material (12) solidified by light irradiation.

7. The method as claimed in any of the preceding claims, **characterized in that** the raw material (12) is not completely solidified by means of the computer-controlled, punctual targeted light irradiation in a first pass.

8. The method as claimed in the preceding claim, **characterized in that** one or more liquid raw material points (9) are applied to a platform (15) and/or to already solidified raw material and are solidified by light irradiation before further points (9) of liquid raw material (12) are applied.

9. The method as claimed in any of the preceding claims, **characterized in that** the liquid raw material (12) is applied in a layer-by-layer manner to a platform (15) and/or to already solidified raw material and is solidified by computer-controlled, targeted light irradiation before a further layer of liquid raw material (12) is applied.

10. The method as claimed in any of the preceding claims, **characterized in that** the component (10) is subjected to a heat after-treatment after complete or partial conclusion of the solidification process for the liquid raw material (12) by computer-controlled, targeted light irradiation.

11. An apparatus for producing a component (10) by means of an additive manufacturing process, comprising:
a) a manufacturing zone (1) at which the component (10) to be produced is formed by means of raw material (12) which is to be arranged there, which is liquid and which is solidifiable by heating,
b) a raw material dispensing unit (2) for dispensing the raw material (12) to the manufacturing zone (1),
c) a light-beam source (3) for emitting a light beam (8),
d) a controllable actuator mechanism (6) which makes it possible to alter the impact point of the light beam (8) of the light-beam source (3) relative to the manufacturing zone (1) in a continuous and/or in a step-by-step manner,
e) a control unit (5) with a computer (16), which control unit is configured to control the actuator mechanism (6),
f) wherein the apparatus is configured to carry out a method for producing a component (10) by means of an additive manufacturing process as claimed in any of the preceding claims,
charcaterized in that the apparatus comprises a preheating unit (13) by means of which the liquid raw material (12) in the raw material dispensing unit is already preheated before dispensing and thus partially solidified.

## Revendications

1. Procédé de fabrication d'un composant (10) au moyen d'un processus de production génératif, le composant (10) étant fabriqué en totalité ou en partie à partir d'un matériau brut liquide (12), le composant (10) étant fabriqué en totalité ou en partie à partir d'un matériau brut liquide (12), solidifiable par chauffage, le matériau brut (12) étant émis sous forme liquide dans une zone de production (1) et étant chauffé par un rayonnement de lumière ciblé, ponctuel, commandé par ordinateur, et étant ainsi solidifié, en modifiant continuellement et/ou par étapes le point d'impact d'un faisceau de lumière (8) d'une source de faisceau de lumière (3) par rapport à la zone de production (1), **caractérisé en ce que** le matériau brut liquide (12) est déjà préchauffé avant l'émission dans le dispositif d'émission de matériau brut au moyen d'un dispositif de préchauffage (13), et ainsi partiellement solidifié.

2. Procédé selon l'une quelconque des revendications précédentes, **caractérisé en ce que** le matériau brut (12) est un matériau polymère.

3. Procédé selon l'une quelconque des revendications précédentes, **caractérisé en ce que** le point d'impact du faisceau de lumière (8) de la source de faisceau de lumière (3) par rapport à la zone de production (1) est asservi à un dispositif d'émission de matériau brut (2), qui émet le matériau brut (12) goutte à goutte, pendant le processus de fabrication du composant (10), de telle sorte que les gouttes de matériau brut (9) fraîchement émises par le dispositif d'émission de matériau brut (2) soient aussitôt chauffées par le faisceau de lumière (8) et ainsi solidifiées.

4. Procédé selon l'une quelconque des revendications précédentes, **caractérisé en ce que** le matériau brut (12) est mélangé avec un matériau favorisant le durcissement.

5. Procédé selon l'une quelconque des revendications précédentes, **caractérisé en ce que** le faisceau de lumière (8) est un faisceau de lumière infrarouge ou comprend au moins majoritairement des fractions spectrales infrarouges.

6. Procédé selon l'une quelconque des revendications précédentes, **caractérisé en ce que** le composant (10) est fabriqué à partir de plusieurs matériaux différents, au moins une structure support pour au moins un élément partiel supplémentaire du composant (10) étant générée à partir du matériau brut (12) solidifié par un rayonnement de lumière.

7. Procédé selon l'une quelconque des revendications précédentes, **caractérisé en ce que** le matériau brut (12) n'est pas solidifié en totalité par le rayonnement de lumière ciblé, ponctuel, commandé par ordinateur, lors d'une première phase de travail.

8. Procédé selon la revendication précédente, **caractérisé en ce qu'**un ou plusieurs points de matériau brut liquide (9) sont appliqués sur une plateforme (15) et/ou sur un matériau brut déjà solidifié, et solidifiés avec un rayonnement de lumière, avant que des points supplémentaires (9) de matériau brut liquide (12) soient appliqués.

9. Procédé selon l'une quelconque des revendications précédentes, **caractérisé en ce que** le matériau brut liquide (12) est appliqué en couches sur une plateforme (15) et/ou un matériau brut déjà solidifié, et solidifié par un rayonnement de lumière ciblé, commandé par ordinateur, avant qu'une couche supplémentaire de matériau brut liquide (12) soit appliquée.

10. Procédé selon l'une quelconque des revendications précédentes, **caractérisé en ce que** le composant (10) est soumis à un post-traitement thermique après l'achèvement total ou partiel du processus de solidification du matériau brut liquide (12) par un rayonnement de lumière ciblé, commandé par ordinateur.

11. Unité pour la fabrication d'un composant (10) au moyen d'un processus de production génératif, comprenant :
a) une zone de production (1), au niveau de laquelle le composant à fabriquer (10) se forme au moyen d'un matériau brut liquide (12) solidifiable par chauffage, à y agencer,
b) un dispositif d'émission de matériau brut (2) pour l'émission du matériau brut (12) au niveau de la zone de production (1),
c) une source de faisceau de lumière (3) pour l'émission d'un faisceau de lumière (8),
d) un mécanisme d'actionnement réglable (6), avec lequel le point d'impact du faisceau de lumière (8) de la source de faisceau de lumière (3) peut être modifié continuellement et/ou par étapes par rapport à la zone de production (1),
e) une unité de commande (5) comprenant un ordinateur (16), qui est conçue pour la commande du mécanisme d'actionnement (6),
f) l'unité étant conçue pour la réalisation d'un procédé de fabrication d'un composant (10) au moyen d'un processus de production génératif selon l'une quelconque des revendications précédentes,
**caractérisée en ce que** l'unité comprend un dispositif de préchauffage (13), par lequel le matériau brut liquide (12) est déjà préchauffé avant l'émission dans le dispositif d'émission de matériau brut, et ainsi partiellement solidifié.
